# EUROPEAN PATENT APPLICATION

(11) **EP 4 450 081 A1**
(43) Date of publication of application: **23.10.2024**
(21) Application number: 22906673.3
(22) Date of filing: 16.12.2022
(51) Int. Cl.: A61K 39/00, A61P 35/00, A61P 37/02

(54) **ANTI-IMMUNOGLOBULIN DEGRADING ENZYME-DIGESTED FC VARIANT**

(30) Priority: 16.12.2021 CN 202111547070
(71) Applicant: Shanghai Bao Pharmaceuticals Co., Ltd., Shanghai 201908 (CN)
(72) Inventor: WANG, Zheng, Shanghai 201908 (CN); XU, Yunxia, Shanghai 201908 (CN); LIU, Yanjun, Shanghai 201908 (CN); LV, Baojie, Shanghai 201908 (CN)
(74) Representative: Bayramoglu et al.
(86) International application number: PCT/CN2022/139441
(87) International publication number: WO 2023/109928

(57) **Abstract**

Provided is a molecule comprising an Fc variant. Compared with a molecule comprising an IgG wild-type Fc, the molecule is resistant to the degradation of an immunoglobulin enzyme, and the molecule comprises a human IgG Fc region having a mutation, the mutation being one or more substitutions or deletions at a G237 position (EU numbering), and the substitution preferably being G237A. Further provided are a nucleic acid encoding the molecule, a vector comprising the nucleic acid and a host cell, and a method for preparing the molecule.

## Description

### Technical Field

The present invention relates to the field of biomedicine and in particular to a human IgG Fc variant and a molecule containing the variant.

### Background Art

Immunoglobulin G (IgG) is the main antibody component of serum, accounting for about 75% of serum immunoglobulin. It plays a major protective role in the body's immunity and can effectively prevent infectious diseases. In addition to having a protective effect, IgG is also associated with diseases. In diseases such as rheumatoid arthritis, systemic lupus erythematosus, myasthenia gravis, and autoimmune (or spontaneous) thrombocytopenia, the IgG antibody reacts with a human self-molecule and IgG causes acute transplant rejection in organ transplants. For some diseases, such as multiple myeloma, 50% of patients have high blood IgG levels, which affect the metabolism and efficacy of a therapeutic antibody.

Research has found that IdeS-type immunoglobulin degrading enzymes can degrade blood IgG, ruling out the negative impact of human IgG on the therapeutic antibody. On the other hand, the half-life of the therapeutic antibody can also be extended by lowering blood IgG levels (Abdallah, H. M. and A. Z. X. Zhu. 2020. Clin Pharmacol Ther 107(2): 423-434). IdeS can completely cleavage human blood IgG within 2 hr after use (Winstedt, L. et al. 2015. PLoS One 10 (7): e0132011), which is an effective method to exclude IgG interference. However, no therapeutic antibody can be used for at least 4 days after administration of immunoglobulin degrading enzymes such as IdeS. Therefore, there is an urgent need for a therapeutic antibody technology that can resist IdeS enzyme cleavage, so that while using IdeS to reduce blood IgG interference, the antibody can be used for treatment, that is, to achieve simultaneous administration of IdeS and the therapeutic antibody.

### Summary of the Invention

The details of one or more embodiments of the invention are set forth in the description below. The other features, objectives, and advantages of the present invention will be apparent in the specification and claims.
1. A molecule comprising an Fc variant, wherein compared with a molecule comprising an IgG wild-type Fc, the molecule is resistant to the degradation of an immunoglobulin enzyme, and the molecule comprises a human IgG Fc region having a mutation, the mutation being one or more substitutions or deletions at a G237 position (EU numbering), and the substitution preferably being G237A; preferably, the human IgG Fc region comprises at least the sequence shown in SEQ ID NO: 19.
2. The molecule of item 1, wherein the Fc molecule is resistant to the degradation of a protease capable of cleaving a molecule comprising an IgG wild-type Fc between residues 236-237 (EU numbering).
3. The molecule of item 1 or 2, wherein compared with the wild-type IgG, the molecule is resistant to the degradation of an enzyme selected from the group consisting of IdeS, IdeZ, IdeE, IdeZ2, IdeE2, IdeSORK, Xork, and variants thereof.
4. The molecule of any one of items 1-3, wherein the IgG of the molecule is selected from the group consisting of IgG1, IgG2, IgG3, and IgG4.
5. The molecule of any one of items 1-4, wherein compared with a molecule comprising an IgG wild-type Fc, the molecule has promoted or enhanced effector functions selected from the group consisting of ADCC, ADCP, and CDC.
6. The molecule of item 5, further comprising one or more substitutions in the human IgG wild-type Fc region sequence selected from the group consisting of S239D, I332E, G236A, S298A/E333A/K334A, S239D/I332E, S239D/A330L/I332E, G236A/S239D/I332E, G236A/A330L/I332E, G236A/S239D/A330L/I332E, F243L/R292P/Y300L, F243L/R292P/Y300L/V305I/P396L, L235V/F243L/R292P/Y300L/P396L, P247I/A339Q, K326A/E333A, K326W/E333S, E333A/K334A, H268F/S324T/1332E, S239D/H268F/S324T/I332E, S267E/H268F/S324T/I332E, K326A/I332E/E333A, S239D/K326A/E333A and S267E/I332E; preferably, the substitution is selected from the group consisting of S239D, I332E and S239D/I332E.
7. The molecule of any one of items 1-6, further comprising an N-glycan modification having a fucose content of less than 50%, preferably less than 10%.
8. The Fc molecule of any one of items 1-7, further comprising an N-glycan modification having a terminal galactose modification content of higher than 30%, preferably higher than 50%.
9. The molecule of any one of items 1-8, wherein the molecule is an antibody or Fc fusion protein.
10. The molecule of item 9, wherein the antibody binds to an antigen on a virus, bacterium, tumor cell, tumor stroma, or tumor vasculature, preferably the antibody specifically binds to one or two antigens selected from the group consisting of COVID-19, CD38, CD20, FR5, BCMA, SLAM7, CD73, GPRCD5 and CD3, most preferably, the antibody specifically binds to CD38.
11. The molecule of item 9 or 10, wherein the antibody comprises at least one heavy chain and at least one light chain, wherein the heavy chain comprises HCDR1 as shown in SEQ ID NO: 1, HCDR2 as shown in SEQ ID NO: 2, HCDR3 as shown in SEQ ID NO: 3; or, HCDR1 as shown in SEQ ID NO: 4, HCDR2 as shown in SEQ ID NO: 5, and HCDR3 as shown in SEQ ID NO: 6; wherein the light chain comprises LCDR1 as shown in SEQ ID NO: 7, LCDR2 as shown in SEQ ID NO: 8, and LCDR3 as shown in SEQ ID NO: 9; or, LCDR1 as shown in SEQ ID NO: 10, LCDR2 as shown in SEQ ID NO: 11, and LCDR3 as shown in SEQ ID NO: 12.
12. The molecule of any one of items 9-11, wherein the antibody comprises at least one heavy chain and at least one light chain, wherein the heavy chain comprises a heavy chain variable region as shown in an amino acid sequence of SEQ ID NO: 13 and the light chain comprises a light chain variable region as shown in an amino acid sequence of SEQ ID NO: 14; or wherein the heavy chain comprises a heavy chain variable region as shown in an amino acid sequence of SEQ ID NO: 15 and the light chain comprises a light chain variable region as shown in an amino acid sequence of SEQ ID NO: 16.
13. The molecule of any one of items 9-12, wherein an epitope-binding fragment of the antibody is Fab, Fab' and F(ab')2, scFv or a disulfide-linked Fv fragment.
14. The molecule of any one of items 1-4, wherein compared with a molecule comprising an IgG1 wild-type Fc, the molecule has weakened and/or eliminated effector functions selected from the group consisting of ADCC, ADCP, and CDC.
15. The Fc molecule of item 14, further comprising one or more substitutions in the human IgG1 wild-type Fc sequence selected from the group consisting of N297A, N297Q, N297G, L235A, L235A/E318A, L234A/L235A, G236R/L328R, S298G/T299A, L234F/L235E/P331S, E233P/L234V/L235A/G236del/S267K, L234A/L235A/P329G, and L234F/L235E/D265A; preferably, the substitution is selected from the group consisting of N297A, N297Q, N297G, L234A/L235A, L235A, and L234F/L235E/P331S.
16. The molecule of any one of items 1-15, wherein compared with a molecule comprising an IgG1 wild-type Fc, the molecule has increased affinity to FcRn.
17. The molecule of item 16, further comprising one or more substitutions in the sequence of IgG1 wild-type Fc selected from the group consisting of YTEKF, YTEKF+V264A, and YTEKF+V264E.
18. An isolated binding polypeptide molecule comprising the following: (i) a binding domain capable of binding to a target molecule on or bound to a cell, and (ii) an Fc domain having amino acid sequences of CH2 and CH3 constant domains of a human IgG1 heavy chain, with residue G237 as defined by the EU numbering system being substituted or deleted; characterized in that the binding molecule is capable of binding to a target molecule on a target cell and the molecule produces a measurable CDC activity, or a necessary effector cell-mediated target cell damaging activity, preferably wherein the substitution at G237 is G237A.
19. A nucleic acid encoding the molecule of any one of items 1-17 or the binding polypeptide molecule of item 18.
20. An expression vector comprising the nucleic acid of item 19.
21. A host cell comprising the nucleic acid of item 19 or the expression vector of item 20.
22. A method for preparing the molecule of any one of items 1-17 or the binding polypeptide molecule of item 18, comprising:
   (a) providing the host cell of item 21;
   (b) culturing the host cell under conditions suitable for the expression of the molecule or the binding polypeptide molecule; and
   (b) optionally recovering the molecule or the binding polypeptide molecule from the culture of step (b).

### Brief Description of the Drawings

FIG. 1 shows the immunoglobulin degrading enzyme degrading mutant antibody. M is a molecular weight marker; 1-5: antibodies 1-3, 5, and 6; 6-13: cleaved antibodies 1-3, 5, 6, wild type, wild type, and 7. IgG is a full-length antibody, scIgG is an antibody with one Fc cleaved, F(ab)2 is a cleaved Fab dimer, and Fc is a cleaved Fc monomer.
FIGs. 2A, 2B, and 2C show the ADCC activities of different mutant antibodies.
FIG. 3 shows the ADCP activities of different mutant antibodies.
FIG. 4 shows the CDC activities of different mutant antibodies.
FIG. 5 shows the apoptosis-inducing activities of different mutant antibodies.
FIG. 6 shows the inhibition rates of CD38 enzymatic activity by different mutant antibodies.
FIG. 7 shows the inhibitory effects of different antibodies on xenograft models, Ab being the administered antibody.

### Detailed Description

In view of the deficiencies of the prior art, it is an object of the present invention to provide an Fc mutation that is resistant to cleavage by IdeS-type immunoglobulin-degrading enzymes. The IgG, Fc fusion protein, and Fc fragment comprising the modified mutants can resist the cleavage of IdeS-like immunoglobulin degrading enzymes, and the single-site mutation can achieve the effect of resisting cleavage.

The object of the present invention is achieved by the following technical solution:
1. Definitions
   "Fc", or "Fc-containing protein" refers to a monomeric, dimeric, or heterodimeric protein having at least one immunoglobulin CH2 and CH3 region. The CH2 and CH3 domains may form at least a portion of a dimeric region of a protein (e.g. an antibody). Similar domains of other IgG subclasses can be determined by comparing the amino acid sequences of heavy chains or heavy chain fragments of IgG subclasses with the amino acid sequences of human IgG1, as shown on the website (http://www.imgt.org/IMGTScientificChart/Numbering/ Hu_IGHGnber.html). As used herein, "EU numbering" refers to the numbering of human IgG1 antibody residues. Amino acid positions herein are all labeled with the "EU numbering".

The term "wild-type Fc" refers to a reference polypeptide in the wild-type Fc region or a variant optionally comprising mutations other than those considered. The term "wild-type" or "WT" herein refers to an amino acid sequence or nucleotide sequence found in nature, i.e. it is of natural origin (including allelic variation) and has not been intentionally modified by molecular biological techniques such as mutagenesis (Vidarsson, G. et al. (2014). Front Immunol 5: 520). For example, the "wild-type Fc region" specifically refers to the IgG1 Fc regions with SEQ ID NO: 17 (G1m1, 17 isoform) and SEQ ID NO: 18 (G1m3 isoform).

The term "half-life" refers to the amount of time that an Fc fragment, once present in the serum of a patient to whom it is administered, is eliminated in half from the circulation or other tissues.

The term "Fey Receptor" or "FcyR" refers to an IgG-type immunoglobulin receptor of CD64 (FcyRI), CD32 (FcyRII), and CD16 (FcyRIII), particularly the five expressed receptors (FcyRIa, FcyRIIa, FcyRIIb, FcyRIIIa, FcyRIIIb). Except for human FcyRIIb (which is a receptor that inhibits immune cell activation), they are all effector cell activation receptors (Muta T et al. Nature, 1994, 368: 70-73).

The term "effector cell" refers to any cell bearing an Fc receptor, such as a lymphocyte, a monocyte, a neutrophil, a natural killer (NK) cell, an eosinophil, a basophil, a mast cell, a dendritic cell, a Langerhans cell, and a platelet.

The term "ADCC" refers to antibody-dependent cellular cytotoxicity, an antibody-mediated cellular immune response in which an effector cell expressing Fc receptors (FcR) recognizes a bound antibody on a target cell and subsequently causes lysis of the target cell. NK cells, the primary cells mediating ADCC, express only FcyR III, whereas monocytes express FcyRI, FcyRII, and FcyRIII. The detection methods of ADCC include PBMC, NK cell method, NK92 reporter gene method, and Jurkat reporter gene method. ADCC is measured in the presence of blood mononuclear cells. The detection methods of ADCC include PBMC, NK cell method, NK92 reporter gene method, and Jurkat reporter gene method. ADCC is measured in the presence of blood mononuclear cells.

The term "ADCP" refers to antibody-dependent phagocytosis, a process whereby antibody-coated cells are internalized (fully or partially) by phagocytic immune cells (e.g. macrophages, neutrophils, and dendritic cells) that bind to the Fc effector domain of an Ig. The detection methods of ADCP include the PBMC method, NK92 reporter gene method, and Jurkat reporter gene method. ADCP can also be detected in the presence of a CD14 pos and/or CD11b pos blood monocyte.

The term "C1q" refers to the initiating protein in the classical activation pathway of complement pathways, which is activated by directly binding to the cell surface or antibodies bound to the cell surface through the complement protein C1q. It also plays a role in regulating the phagocytosis of apoptotic cells, activating endothelial cells, and regulating T and B cells.

The term "complement-dependent cytotoxicity" or CDC refers to a cell death-inducing mechanism in which the Fc effector domain of a target-bound antibody activates a series of enzymatic reactions leading to the formation of a hole in the target cell membrane. Generally, antigen-antibody complexes such as those on antibody-coated target cells bind to and activate the complement component Clq, which in turn activates the complement cascade leading to target cell death. Activation of complement may also result in the deposition of complement components on the target cell surface, contributing to ADCC by binding to complement receptors (such as CRB) on leukocytes. The detection method of CDC, such as co-incubation of complement from human blood and target cells, detects the killing of target cells.

The terms "immunoglobulin degrading enzyme" and "IdeS class immunoglobulin degrading enzyme" refer to an enzyme that can cleave the immunoglobulins. The immunoglobulin degrading enzyme herein specifically refers to an IgG-specific degrading protease with a cleavage site between IgG positions G236 and G237 (EU number). Currently known sources include IdeZ, IdeZ2, IdeE, and IdeE2 from Streptococcus equi (Lannergard, J. and B. Guss. 2006. FEMS Microbiol Lett 262(2): 230-235; Hulting, G. et al., (2009). FEMS Microbiol Lett 298(1): 44-50), IdeS from *Streptococcus pyogenes* (Wenig, K. et al., (2004). Proc Natl Acad Sci U S A 101(50): 17371-17376), IdeSORK from *Streptococcus krosus.* They specifically cleave immunoglobulin G (IgG) 1-4. Other immunoglobulin classes such as IgA, IgD, IgE, and IgM are not cleaved. These immunoglobulin-degrading enzymes cleave human IgG into F(ab')2 fragments and Fc fragments between G236 and G237 in the CH2 region. These variants of known enzymes can also play similar roles, as stated in CN107532158A, CN107532156A, WO2022223818A1, WO2021254479A1, and CN115443288A.

The terms "N-glycan", "N-linked glycan", "glycoprotein", "glycosylation", and "glycoform" refer to N-linked oligosaccharides, e.g. oligosaccharides linked to an asparagine residue of a polypeptide via an asparagine-N-acetylglucosamine linkage. The primary sugars found on glycoproteins are glucose, galactose, mannose, fucose, N-acetylgalactosamine (GalNAc), N-acetylglucosamine (GlcNAc), and sialic acid (SA, including NANA, NGNA, and derivatives and analogs thereof, including acetylated NANA or acetylated NGNA). In sugar-engineered Pichia, sialic acid is only N-acetylneuraminic acid (NANA) (Hamilton et al., Science 313 (5792), 1441-1443, 2006). N-glycans have a common pentasaccharide core, i.e. Man3GlcNAc2, where "Man" refers to mannose, "Glc" refers to glucose, "NAc" refers to N-acetyl, and GlcNAc refers to N-acetylglucosamine. N-glycans differ in the number of branches (antennae) contained in the peripheral sugars (e.g. GlcNAc, galactose, fucose, and sialic acid) added to the Man3GlcNAc2 ("Man3") core structure, also referred to as "trimannose cores", "pentasaccharide cores" or "oligomannose cores". N-glycans are classified according to their branching components (e.g. high mannose, complexes, or hybrids). An N-glycan terminus can be modified with up to four sialic acids.

The term "G0" refers to a complex bidirectional antennal oligosaccharide (GlcNAc₂Man₃GlcNAc₂) without galactose or fucose; "G1" refers to a complex bidirectional antennal oligosaccharide (GalGlcNAc₂Man₃GlcNAc₂) without fucose and containing one galactose residue; "G2" refers to a complex bidirectional antennal oligosaccharide (Gal₂GlcNAc₂Man₃GlcNAc₂) without fucose and containing two galactose residues; "G0F" refers to a complex bidirectional antennal oligosaccharide (GlcNAc₂Man₃GlcNAc₂F) containing core fucose and without galactose; "G1F" refers to a complex bidirectional antennal oligosaccharide (GalGlcNAc₂Man₃GlcNAc₂F) containing one core fucose and one galactose residue; and "G2F" refers to a complex bidirectional antennal oligosaccharide (Gal₂GlcNAc₂Man₃GlcNAc₂F) containing core fucose and two galactose residues.

The term "hybrid" N-glycan refers to an N-glycan having at least one GlcNAc at the end of the 1,3-mannose arm of the trimannose core and zero or more one mannose on 1 position. The 6 mannose arms of the trimannose core.

IgG is the major serum immunoglobulin in blood, and the blood IgG level in healthy people is about 10 g/L. It is a glycoprotein comprising of two identical heavy and two light chains, which in turn comprised of a variable region and a constant region. IgG comprises a single, N-linked glycan at position Asn297 of the CH2 domain of each Fc region of its two heavy chains. The covalently linked, complex carbohydrate is comprised of a core, double-branched penta-polysaccharide comprising N-acetylglucosamine (GlcNAc) and mannose. Glycosylation modifications of Fc are involved in antibody function. Among them, the knock-out of fucose can greatly enhance the IgG modification of the antibody. Modification of the terminal galactose increases CDC function.

The term "immunoconjugate" refers to a molecule obtained by conjugating one or more therapeutic agents, such as a cytotoxic agent, a chemotherapeutic agent, a drug, a growth inhibitor, a toxin (e.g. a protein toxin, an enzymatically active toxin of bacterial, fungal, plant, or animal origin, or a fragment thereof), or a radioisotope, to a variant of the invention.

The term "resisting cleavage", or "resistant" to a protease, refers to an immunoglobulin IgG or Fc immunoglobulin antibody fragment structure having the property of not being cleaved by an immunoglobulin degrading enzyme as described above.

The term "vector" or "expression vector" used herein refers to a vector used according to the present invention as a vehicle for introducing a desired gene into a cell and expressing the desired gene in the cell. One skilled in the art will recognize that such vectors can be readily selected from plasmids, phages, viruses, and retroviruses. In general, vectors suitable for the present invention will contain selection markers, suitable restriction sites to facilitate the cloning of the desired gene, and the ability to enter and/or replicate in eukaryotic or prokaryotic cells.

As used herein, the term "transformation" shall be used in a broad sense to refer to the introduction of DNA into a recipient host cell, thereby changing the genotype and thereby causing a change in the recipient cell.

Similarly, a "host cell" refers to a cell transformed with a vector constructed using recombinant DNA technology and encoding at least one heterologous gene. In describing methods for isolating polypeptides from recombinant hosts, the terms "cell" and "cell culture" are used interchangeably to refer to the source of the Fc variant, unless specifically indicated otherwise. In other words, recovery of an Fc variant from a "cell" can mean recovery from a settled whole cell, or from a cell culture containing both medium and suspended cells.

### 2. Mutation resisting cleavage

The Fc variant of the present invention comprising the G237 mutation (EU numbering) is not degraded by cleavage under the coexistence of the immunoglobulin degrading enzyme as described above. The cleavage of immunoglobulin degrading enzymes is structure specific and requires at least the CH2 domain to be present (Wenig, K. et al., 2004. Proc Natl Acad Sci U S A 101(50): 17371-17376). The CH2 domain needs to contain at least the sequence from L235 such as SEQ ID NO: 19 (Novarra, S. et al. 2016. MAbs 8(6): 1118-1125). Therefore, the Fc variant of the present invention comprises at least a CH2 region having a G237 mutation. Where the G237 mutation may be one or more substitutions or deletions at this position, the substitution is preferably G237A.

The present invention first discovered that only G237 single point mutation can achieve the effect of resisting the cleavage of the immunoglobulin degrading enzyme. The only report that can achieve this effect (CN103260640B) has a complex mutation combination, which involves substituting IgG1CH2 region E233-L234-L235-G236 with P233-V234-A235 of IgG2, and then combining S239D/I332E or G237A/S239D/I332E.

IgG isoforms other than IgG1, such as IgG2, IgG3, and IgG4, can be degraded by immunoglobulin degrading enzymes (Wenig, K. et al., 2004. Proc Natl Acad Sci U S A 101(50): 17371-17376). In some embodiments described herein, Fc fragments from different IgG subtypes, such as IgG2, IgG3, and IgG4, may be used in the Fc fragments of the present invention. Wild Fc sequences of different allotypes of IgG2-4 (Vidarsson, G., et al. (2014). Front Immunol 5: 520) are resistant to cleavage after substitution or deletion at G237.

In some embodiments described herein, amino acid substitutions may be made to the constant region of an antibody. For example, different IgG1 allotypes, such as the well-known G1m17 allotype, G1m3 allotype, or G1m1 allotype, or combinations thereof, may be used in the Fc fragments of the present invention; alternatively, different IgG2-4 allotypes are used (Vidarsson, G. et al. (2014). Front Immunol 5: 520) or a combination thereof.

### 3. Promotion or enhancement of effector function

ADCC etc. are the key roles of antibodies to fulfill their functions. G237 mutations may result in attenuation or loss of such effector functions (Hezareh, M. et al., 2001. J Virol 75 (24): 12161-12168.). In some embodiments described herein, amino acid substitutions and/or glycosylation modifications may be made to the Fc region of the molecule to increase the decrease in effector function resulting from the G237 mutation. Compared with a molecule comprising an IgG1 wild-type Fc, the molecule has promoted or enhanced effector functions selected from the group consisting of ADCC, ADCP, and CDC.

In some embodiments, the amino acid substitution made to the Fc region comprises one or more substitutions in the wild-type human IgG1 sequence selected from the group consisting of S239D, I332E, G236A, S298A/E333A/K334A, S239D/I332E, S239D/A330L/I332E, G236A/S239D/I332E, G236A/A330L/I332E, G236A/S239D/A330L/I332E, F243L/R292P/Y300L, F243L/R292P/Y300L/V305I/P396L, L235V/F243L/R292P/Y300L/P396L, P247I/A339Q, K326A/E333A, K326W/E333S, E333A/K334A, H268F/S324T/I332E, S239D/H268F/S324T/I332E, S267E/H268F/S324T/I332E, K326A/I332E/E333A, S239D/K326A/E333A and S267E/I332E; preferably, the substitution is I332E, S239D/I332E.

In some embodiments, the glycosylation modification of the Fc region is characterized by including N-glycan modification with reduced fucose modification, wherein the fucose content in the N-glycan modification is less than 50%, preferably less than 10%. Reduced fucose modification may increase antibody ADCC activity.

In some embodiments, the glycosylation modification of the Fc region is characterized by including N-glycan modifications with increased content of terminal galactose modification, wherein the content of the terminal galactose modification in the N-glycan modification is higher than 30%, preferably higher than 50%. Increased terminal galactose modification can increase the antibody CDC activity.

### 4. Reduction or elimination of effector function

In some embodiments, an amino acid substitution is made to the Fc region such that compared with the molecule containing IgG1 wild-type Fc, the molecule has weakened and/or eliminated effector functions selected from the group consisting of ADCC, ADCP, and CDC. The substitution is selected from the group consisting of N297A, N297Q, N297G, L235A, L235A/E318A, L234A/L235A, G236R/L328R, S298G/T299A, L234F/L235E/P331S, E233P/L234V/L235A/G236del/S267K, L234A/L235A/P329G, and L234F/L235E/D265A; preferably, the substitution is selected from the group consisting of N297A, N297Q, N297G, L234A/L235A, L235A, and L234F/L235E/P331S.

### 5. Increased FcRn binding

The affinity of Fc and FcRn regulates antibody metabolism in humans. In some embodiments, the Fc variant comprises a mutation that increases the affinity of Fc to FcRn, wherein compared to a molecule comprising an IgG1 wild-type Fc, the molecule has increased affinity to FcRn. The mutation is preferably N434A, N434W, M252Y/S254T/T256E, M428L/N434S, M252Y/S254T/T256E/H433K/N434F, T250Q/M428L, T307Q/N434A, M252Y/V308P/N434Y, T307P/L309Q/Q311R, H285D/T307Q/A378V, L309D/Q311H/N434S, M252Y/T256D, T256D/T307Q, T256D/T307W.

Other Fc variants used in the present invention include, but are not limited to, the mutations described by Dall'Acqua et al. (WF, D. A. et al., 2002. Journal of immunology. 169(9): 5171-5180), mutations described by Shan et al. (Shan, L. et al., (2016). PLoS One 11(8): e0160345.), mutations described by Lee et al., (Lee, C. H. et al., (2019). Nat Commun 10(1): 5031.), mutations described by Mackness et al., (Mackness, B. C. et al., (2019). MAbs 11 (7): 1276-1288.), mutations described by Christophe et al., (Dumet Christophe, Pottier Jérémy, Gouilleux-Gruart Valérie et al., MAbs, 2019, 11: 1341-1350).

In some embodiments, the Fc molecule further comprises one or more substitutions in the wild-type human IgG1 sequence selected from the group consisting of YTEKF, YTEKF+V264A, and YTEKF+V264E.

In some embodiments, the glycosylation modification of the Fc region is characterized by including N-glycan modifications and having increased terminal sialic acid modifications, wherein the sialic acid content is higher than 20%, preferably higher than 30%. Substitution of some amino acids in the Fc region can increase the level of sialic acid modification. The preferred amino acid substitutions are F241A, F243A, V262E, V264A, V264E, E293DEL, E294DEL, and Y300A.

### 6. Antigen binding domain

The molecules of the invention are resistant to immunoglobulin-degrading enzymes, which allow for structure-specific cleavage and require at least the CH2 domain to be included (Wenig, K. et al., 2004. Proc Natl Acad Sci U S A 101(50): 17371-17376). The CH2 domain needs to comprise at least the sequence from L235 such as SEQ ID NO: 19 (Novarra, S. et al. 2016. MAbs 8(6): 1118-1125). The invention therefore encompasses any antibody or fusion protein comprising a human Fc CH2 domain. Where the antibody further has an altered effector function, for example, increased ADCC, ADCP, and/or CDC. In some embodiments, it can also be a reduced effect function.

The antibody binding domain can be generated using methods known to those skilled in the art. Structures of antibody binding regions include but are not limited to, Fab, Fab', F(ab')2, scFv, VHH, heavy chain variable region, light chain variable region, CDRs, and the like.

The molecules of the invention comprise a targeting moiety. The targeting moiety is selected from a tumor cell surface antigen, an enzyme, a hormone, a receptor, a cytokine, an immune cell surface antigen, an adhesion molecule, and the like. The targeting moiety may also have non-proteinaceous properties, such as carbohydrates, lipids, lipopolysaccharides, organic molecules, or metals or metal complexes. Typically, when present, the targeting molecule will be linked to the Fc through a linker, which may be a polypeptide or a non-polypeptide.

The molecules of the invention comprise a targeting moiety, wherein the targeting moiety binds to an antigen on a virus, bacterium, tumor cell, tumor stroma, or tumor vasculature. Preferably, the targeting moiety specifically binds to one or two antigens selected from the group consisting of COVID-19, CD38, CD20, FR5, BCMA, SLAM7, CD73, GPRCD5, and CD3. Most preferably, the antibody specifically binds to CD38.

In an embodiment, the targeting moiety of the invention comprises at least one heavy chain and at least one light chain. Wherein the heavy chain comprises three complementarity determining regions as represented by the amino acid sequences of SEQ ID NO: 1, SEQ ID NO: 2, and SEQ ID NO: 3, and the light chain comprises three complementarity determining regions as represented by the amino acid sequences of SEQ ID NO: 7, SEQ ID NO: 8, and SEQ ID NO: 9.

In an embodiment, the heavy chain comprises a variable region as shown in SEQ ID NO: 13; the light chain comprises a variable region as shown in SEQ ID NO: 15. Preferably, it comprises a light chain as shown in SEQ ID NO: 28 and a heavy chain as shown in SEQ ID NO: 30;
In an embodiment, the targeting moiety of the invention comprises at least one heavy chain and at least one light chain. Wherein the heavy chain comprises three complementarity determining regions as represented by the amino acid sequences of SEQ ID NO: 4, SEQ ID NO: 5, and SEQ ID NO: 6, and the light chain comprises three complementarity determining regions as represented by the amino acid sequences of SEQ ID NO: 10, SEQ ID NO: 11, and SEQ ID NO: 12.

In an embodiment, the heavy chain comprises a variable region as shown in SEQ ID NO: 14; the light chain comprises a variable region as shown in SEQ ID NO: 16. Preferably, it comprises a light chain as shown in SEQ ID NO: 27, and a heavy chain as shown in SEQ ID NO: 23 or SEQ ID NO: 25.

### 7. Preparation method

In an embodiment, the invention provides an isolated nucleic acid encoding an Fc variant of the invention; a vector comprising the nucleic acid; and a host cell comprising the nucleic acid and/or vector. Polynucleotides encoding the Fc variants disclosed herein are typically inserted into expression vectors for introduction into host cells that can be used to produce desired amounts of the claimed Fc variants, i.e. the Fc variants of the present invention. Thus, in certain aspects, the present invention provides expression vectors comprising the polynucleotides disclosed herein, as well as host cells comprising these expression vectors and polynucleotides.

The present invention also provides a method for preparing the G236A antibody. The method comprises providing an isolated nucleic acid encoding a sequence of a polypeptide as described above; an expression vector comprising a nucleic acid; and a host cell comprising a nucleic acid. The invention is also characterized by a method for producing a polypeptide. The method includes culturing a host cell in a culture medium under conditions that permit the expression of a polypeptide encoded by the nucleic acid and purifying the polypeptide from the cultured cell or the culture medium of the cell.

A variety of expression vector systems may be used for the purposes of the present invention. For example, one class of vectors utilizes DNA elements derived from animal viruses such as bovine papillomavirus, polyoma virus, adenovirus, vaccinia virus, baculovirus, retrovirus (RSV, MMTV, or MOMLV), or SV40 virus. Others involve the use of polycistronic systems with internal ribosome binding sites. In addition, cells that have integrated DNA into their chromosomes can be selected by introducing one or more biomarkers that allow for the selection of transfected host cells. The marker may confer prototrophy to an auxotrophic host, confer biocide resistance (e.g. antibiotics), or resistance to heavy metals (e.g. copper). The selectable marker gene may be directly linked to the DNA sequence to be expressed or introduced into the same cell by co-transformation. The optimal synthesis of mRNA may also require other elements. These elements may include signal sequences, splicing signals, and transcriptional promoter, enhancer, and termination signals.

More generally, once the vector or DNA sequence encoding the Fc variant has been prepared, the expression vector may be introduced into a suitable host cell. That is, it can transform host cells. The introduction of the plasmid into the host cell can be accomplished by a variety of techniques known to those skilled in the art. These techniques include but are not limited to, transfection (including electrophoresis and electroporation), protoplast fusion, calcium phosphate precipitation, cell fusion with enveloped DNA, microinjection, and infection with whole viruses. More preferably, the plasmid is introduced into the host by electroporation. The transformed cells are cultured under conditions suitable for production of the Fc variant and the expression of the Fc variant is determined. Exemplary assay techniques include enzyme-linked immunosorbent assay (ELISA), surface plasmon resonance (SPR), fluorescence-activated cell sorter analysis (FACS), immunohistochemistry, and the like.

In an embodiment, the invention provides a method for preparing an Fc variant of the invention, the method comprising: mutating a wild-type Fc fragment of wild-type IgG1, the sequence of which is shown in SEQ ID NO: 17 or 18, to obtain a corresponding Fc variant.

In an embodiment, the mutation is selected from the group consisting of G237A, G237A/I332E, G237A/I332E/S239D, G237A/M252Y/S254T/T256E, G237A/M428L/N434S, G237A/M252Y/S254T/T256E/H433K/N434F.

In an embodiment, the Fc variant is obtained by:
(i) introducing a nucleic acid comprising a nucleic acid encoding an Fc variant of the present invention into a host cell;
(ii) culturing the host cell in a culture medium under conditions permitting expression of the Fc variant of the present invention; and,
(iii) purifying the Fc variant of the present invention from cultured cells or the culture medium of cells.

In an embodiment, the host cell does not produce immunogenic NGNA and α-galactose.

In an embodiment, the host cell line used to express an Fc variant of the invention is derived from a mammal; one skilled in the art can determine the particular host cell best suited for expressing the desired gene product. Exemplary host cell lines include but are not limited to, DG44 and DUXB11 (Chinese hamster ovary cell line, DHFR⁻), SP2/0 (mouse myeloma), and 293 (human kidney). In an embodiment, the cell line has altered glycosylation, e.g. fucoidan-free glycosylation, of an Fc variant expressed therefrom (e.g. PER. C6 or CHO cell lines with FUT8 knockout). In an embodiment, NS0 cells can be used. CHO cells are particularly preferred. Host cell lines are generally available from the commercial services American Tissue Culture Collection or from the public literature.

*In vitro* production allows scale-up to obtain large numbers of the desired Fc variants. Techniques for mammalian cell culture are known in the art and include homogeneous suspension culture, e.g. in a bioreactor, or immobilized or entrapped cell culture, e.g. in a hollow fiber, microcapsule, or on agarose microbead. If necessary and/or desired, the solution of the Fc variant may be purified by conventional purification methods (e.g. gel filtration, affinity chromatography, ion exchange chromatography, hydrophobic chromatography, etc.).

Genes encoding Fc variants of the invention can also be expressed in non-mammalian cells (e.g. yeast or plant cells). The most commonly used of eukaryotic microorganisms are *accharomyces cerevisiae* or common baker's yeast, but a variety of other strains are commonly available.

In addition to cell-based expression systems, cell-free or chemical synthesis methods can be used to generate Fc variants. In certain embodiments, Fc variants are produced by in vitro chemical synthesis.

### 8. Pharmaceutical composition

The present invention provides a pharmaceutical composition comprising the peptide or nucleic acid as described above.

The invention further provides a pharmaceutical formulation comprising (i) a polypeptide or nucleic acid as described above and/or a pharmaceutical combination as described above, and (ii) an immunoglobulin degrading enzyme. The immunoglobulin degrading enzyme is selected from the group consisting of IdeS, IdeZ, IdeZ2, IdeE, IdeE2, IdeSORK, Xork, and variants thereof. The pharmaceutical compositions may be formulated for parenteral administration (e.g. intravenous or intramuscular) by bolus injection. The composition may take such forms as a suspension, solution or emulsion in an oily or aqueous vehicle, and may contain formulatory agents such as a suspending, stabilizing and/or dispersing agent. Alternatively, the active ingredient may be in the form of a powder for constitution with a suitable vehicle, e.g. pyrogen-free water.

The present invention also provides an immunoconjugate comprising the variant disclosed herein conjugated (chemically bonded) to one or more therapeutic agents, such as a cytotoxic agent, a chemotherapeutic agent, a drug, a growth inhibitor, a toxin (e.g. a protein toxin, an enzymatically active toxin of bacterial, fungal, plant, or animal origin, or a fragment thereof), or a radioisotope, to a variant of the invention.

### 9. Use of Fc variant and drug combination

The present invention provides a molecule and/or pharmaceutical composition of the invention for use in a method of treating a disease that is cancer or autoimmune disease, wherein the cancer is multiple myeloma.

The present invention provides use of a molecule and/or a pharmaceutical composition of the invention in the manufacture of a drug for the treatment of a disease, in particular cancer or autoimmune disease. The drug may also be used to induce ADCC, ADCP, apoptosis-mediated depletion of CD38-positive cells.

The present invention provides a method of treating an individual suffering from cancer or autoimmune disease, comprising administering to the individual an effective amount of a molecule and/or a pharmaceutical composition of the invention. Further included is administering to a subject in need thereof a therapeutically effective amount of a polypeptide or nucleic acid encoding a polypeptide as described above. Further provided is use of a polypeptide or nucleic acid in the manufacture of a drug for the treatment of an autoimmune disease and/or tumor; preferably, the disease is a CD38-associated cancer or autoimmune disease. The invention also is characterized by an isolated polypeptide, nucleic acid, expression vector, host cell, pharmaceutical combination, or method for treating an autoimmune disease and/or tumor substantially as shown and described herein; preferably, the disease is a CD38-associated cancer or autoimmune disease. The CD38-associated cancer is a solid tumor, a B-cell lymphoma, or multiple myeloma.

The present invention provides a method of ADCC, ADCP-mediated depletion of CD38 positive cells in an individual comprising administering to the individual an effective amount of a molecule and/or pharmaceutical composition of the invention to induce ADCC, ADCP, apoptosis-mediated depletion of CD38 positive cells.

The Fc variant of the invention has the following advantages: 1) resists cleavage of immunoglobulin degrading enzyme; 2) can be used simultaneously with immunoglobulin degrading enzyme; 3) has increased bioavailability when used with the immunoglobulin degrading enzyme.

The preferred embodiments of the present invention can be obtained by any combination of the above-mentioned preferred conditions on the basis of common knowledge in the art.

The reagents and raw materials used in the present invention are commercially available.

### Examples

The invention is further illustrated by way of the following examples, without thereby limiting the invention to the scope of the described examples. The experimental methods in the following embodiments that do not specify specific conditions shall be selected according to conventional methods and conditions, or according to the product manual.

### Example 1: Protein preparation

Sequence synthesis: the base sequences encoding the amino acid sequences of Table 1 except IdeS were synthesized and constructed into a mammalian cell expression vector pcDNA3.1.

Expression and purification: the heavy and light chains were mixed in a 1: 2 ratio and respectively transiently transferred to ExpiCHO-S cells and fucose-free modified CHO cells. The supernatant was harvested from the 6th to 8th days after transfection and the expression supernatant was centrifugated under 9500 rpm for 20 min, followed by collecting the supernatant. The supernatant was purified by using Protein A to control endotoxin to less than 1 EU/mg. Different numbers of heavy chains, their mutants, and light chains were co-expressed to obtain the wild-type antibody, antibodies 1-6, the heavy chains corresponding to SEQ ID NO: 20-26, and the light chain sequence used being SEQ ID NO: 27. Antibody 7 corresponded to light chain SEQ ID NO: 28 and heavy chain SEQ ID NO: 29.

Expression of IdeS enzyme: after codon optimization, the polynucleotide sequence encoding IdeS (SEQ ID NO: 31) was synthesized and the N-terminal signal peptide sequence MRKRCYSTSAAVLAAVTLFVLSVDRGVIA (SEQ ID NO: 32) was added. After synthesis, the sequence was inserted into pET32a expression vector. After sequencing, the recombinant plasmid for expression was obtained. The mutant recombinant plasmids were electrotransformed into E. coli BL21 Star (DE3) and plated on an LB agarose plate containing 100 ug/ml ampicillin. Incubation was performed overnight at 37°C until colonies grew out. Individual colonies were picked and grown overnight in 200 ul of LB medium containing 100 ug/ml ampicillin at 37°C under 250 rpm. The overnight culture was inoculated into 1 ml of LB medium containing 100 ug/ml ampicillin, incubated at 37°C for 4 h, then 0.1 mM IPTG was added, and the incubation was continued overnight at 30°C. Overnight cultures were collected by centrifugation. SDS-PAGE was used to evaluate the concentration of the mutant protein in the mutant expression supernatant. The supernatant was purified sequentially using ion exchange chromatography and hydrophobic chromatography, and the endotoxin was controlled to be less than 1 EU/mg, ultimately obtaining a pure protein.

**Table 1. Amino acid sequences of expressed protein**

| SEQ ID NO: | SEQ Name | SEQ |
|---|---|---|
| 20 | Heavy Chain Wild-type | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLF PPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 21 | Heavy Chain 1 | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGAPSVFLF PPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 22 | Heavy Chain 2 | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGAPDVFLF PPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK |
| | | GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 23 | Heavy Chain 3 | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGAPSVFLF PPKPKDTLMISRTPEVTCVVVDVSHEDPEVKFNWYVDGVEVHNAKTKP REEQYNSTYRVVSVLTVLHQDWLNGKEYKCKVSNKALPAPEEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 24 | Heavy Chain 4 | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPDVFLF PPKPKDTLMISRTPEVTCVVVDVSHEDPEVKFNWYVDGVEVHNAKTKP REEQYNSTYRVVSVLTVLHQDWLNGKEYKCKVSNKALPAPEEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 25 | Heavy Chain 5 | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGAPDVFLF PPKPKDTLMISRTPEVTCVVVDVSHEDPEVKFNWYVDGVEVHNAKTKP REEQYNSTYRVVSVLTVLHQDWLNGKEYKCKVSNKALPAPEEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 26 | Heavy Chain 6 | QVQLVQSGAEVAKPGTSVKLSCKASGYTFTDYWMQWVKQRPGQGLEW IGTIYPGDGDTGYAQKFQGKATLTADKSSKTVYMHLSSLASEDSAVYYC ARGDYYGSNSLDYWGQGTSVTVSSASTKGPSVFPLAPSSKSTSGGTAAL GCLVKDYFPEPVTVSWNSGALTSGVHTFPAVLQSSGLYSLSSVVTVPSSSL GTQTYICNVNHKPSNTKVDKKVEPKSCDKTHTCPPCPAPELLAGPDVFLF PPKPKDTLMISRTPEVTCVVVDVSHEDPEVKFNWYVDGVEVHNAKTKP REEQYNSTYRVVSVLTVLHQDWLNGKEYKCKVSNKALPAPEEKTISKAK GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSP |
| 27 | Light Chain 1 | DIVMTQSHLSMSTSLGDPVSITCKASQDVSTVVAWYQQKPGQSPRRLIYS ASYRYIGVPDRFTGSGAGTDFTFTISSVQAEDLAVYYCQQHYSPPYTFGG GTKLEIKRTVAAPSVFIFPPSDEQLKSGTASVVCLLNNFYPREAKVQWKV |
| | | DNALQSGNSQESVTEQDSKDSTYSLSSTLTLSKADYEKHKVYACEVTHQ GLSSPVTKSFNRGEC |
| 28 | Light Chain 2 | EIVLTQSPATLSLSPGERATLSCRASQSVSSYLAWYQQKPGQAPRLLIYDA SNRATGIPARFSGSGSGTDFTLTISSLEPEDFAVYYCQQRSNWPPTFGQGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGL SSPVTKSFNRGEC |
| 29 | Heavy Chain 7 | EVQLLESGGGLVQPGGSLRLSCAVSGFTFNSFAMSWVRQAPGKGLEWVS AISGSGGGTYYADSVKGRFTISRDNSKNTLYLQMNSLRAEDTAVYFCAK DKILWFGEPVFDYWGQGTLVTVSSASTKGPSVFPLAPSSKSTSGGTAALG CLVKDYFPEPVTVSWNSGALTSGVHTFPAVLQSSGLYSLSSVVTVPSSSLG TQTYICNVNHKPSNTKVDKRVEPKSCDKTHTCPPCPAPELLGAPSVFLFP PKPKDTLMISRTPEVTCVVVDVSHEDPEVKFNWYVDGVEVHNAKTKPR EEQYNSTYRVVSVLTVLHQDWLNGKEYKCKVSNKALPAPEEKTISKAK GQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQ KSLSLSP |
| 31 | IdeS | MDSFSANQEIRYSEVTPYHVTSVWTKGVTPPANFTQGEDVFHAPYVANQ GWYDITKTFNGKDDLLCGAATAGNMLHWWFDQNKDQIKRYLEEHPEK QKINFNGEQMFDVKEAIDTKNHQLDSKLFEYFKEKAFPYLSTKHLGVFP DHVIDMFINGYRLSLTNHGPTPVKEGSKDPRGGIFDAVFTRGDQSKLLTS RHDFKEKNLKEISDLIKKELTEGKALGLSHTYANVRINHVINLWGADFDS NGNLKAIYVTDSDSNASIGMKKYFVGVNSAGKVAISAKEIKEDNIGAQV LGLFTLSTGQDSWNQTN |

### Example 2. Cleavage Resistance Assay of Mutant

The effect of cleavage of different mutants by IdeS was evaluated by SDS-PAGE. The cleavage reaction was started by adding IdeS at a 1: 50 ratio to 50 ul of a reaction system containing 2 mg/ml of the different variants at 37°C for 30 min. The samples were mixed with an equal volume of non-reducing 2 × SDS loading buffer followed by a 75°C water bath for 5 min. The cleavage products were detected by SDS-PAGE.

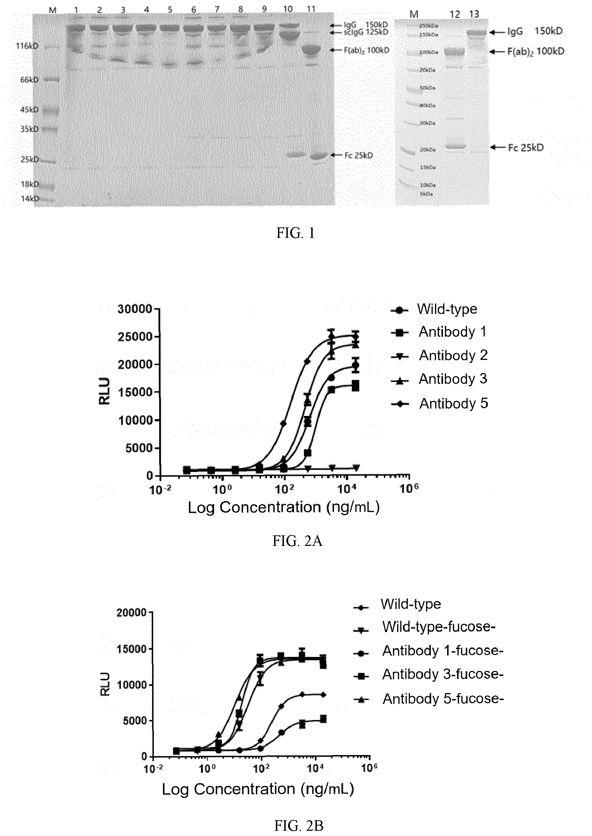

The results of cleavage (FIG. 1) showed that antibodies 1-3, 5, and 7 were all resistant to cleavage; antibody 6 was partially resistant to cleavage; and wild type was not resistant to cleavage.

### Example 3. ADCC activity assay

The ADCC activity of each variant was detected by the reporter gene cell method and PBMC method. Reporter gene methods were performed using Daudi cells at an adjusted density of 1 × 10⁶ cells/ml and plated to a 96-well plate at 25 µL per well. The antibody to be tested was diluted with 1640 culture medium to 20 µg/mL, and then diluted at 6-fold gradient-wise for 7 times to obtain drugs with 8 concentration gradients. 25 µL of the diluted drug was added to the cells of the 96-well plate. The 1640 medium was used as a negative control (NC). The 96-well plates were incubated in a 37°C carbon dioxide incubator for 45 minutes. Reporter gene cells were added and incubated for 6 hours in a carbon dioxide incubator. At the end of incubation, 75 µl of firefly luciferase substrate solution (Rhinogen^{®}) was added per well. The plate was shaken well with a microplate shaker and left at room temperature for 5 min. The value of the relative light unit (RLU) was read with a microplate reader, and the data were analyzed by selecting a four-parameter equation regression model using the Graphpad software.

The PBMC method included: Calcein-AM (Beyotime, cat. C2012), and Daudi cells were co-incubated for 20 minutes to allow the Calcein-AM dye to enter target cells. The use final concentration of Calcein was 4 µM. The incubated cells were seeded into a 96-well plate at 2 × 10⁴ cells/well. The antibody to be tested was diluted with the 1640 medium to a concentration range of 2000-0.02 ng/ml and added to a 96-well plate. 5 × 10⁵ PBMC cells were added per well to obtain a final ratio of PBMC cells to Daudi cells of 25: 1. The 1640 medium was used as a negative control (NC), total control. After incubation at 37°C for 4 hours in a humidified incubator, the cells were centrifuged and the supernatant was taken. The fluorescence value was measured with 490 nm as the excitation wavelength and 515 nm as the emission wavelength. The total control was not centrifuged, and the cells were lysed with 0.1% Triton X-100 directly. After centrifugation, the supernatant was taken for determination of fluorescence. From the fluorescence values, the target cell lysis rate was calculated.

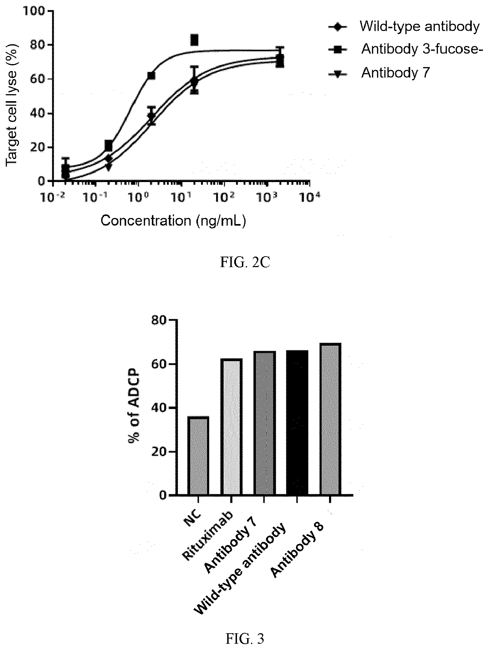

The results show (FIG. 2A) that antibody 1 had reduced ADCC activity relative to the wild type. Antibody 2 had no ADCC activity and antibodies 3 and 5 had enhanced ADCC activity. By comparison of EC50 values, the fold activities of antibodies 1, 3, and 5 relative to the wild type were 0.6, 1.3, and 3.9 fold, respectively. In the antibody sample without Fucose modification (FIG. 2B), the fold activities of antibody 3-Fucose⁻ and antibody 5-Fucose⁻ relative to the wild type were 12.7 and 23.2, respectively, much higher than that of the control by comparison of EC50 values. In the PBMC method, the EC50 values of the wild-type antibody, antibody 3-Fucose⁻ and antibody 7 were 2.072 ng/ml, 0.6578 ng/ml, and 2.095 ng/ml, respectively (FIG. 2C), and the fold activities of antibody 3-Fucose⁻ and antibody 7 relative to the wild-type were 3.1 and 1.0, respectively. Antibody 3-Fucose⁻ is subsequently indicated as antibody 8 for description.

### Example 4. Mutant Stability Assay

The high-throughput protein stability assay system Uncle from Unchained Labs was used to detect the thermostability of the antibody. 1.0 mg/ml of purified mutant sample was loaded for on-line detection: the Tm & Tagg with optional DLS program was used, the temperature interval was set to 25-95°C, and one duplicate well was set for the sample. The thermostability was analyzed by analyzing the resulting parameters Tm (protein melting temperature) and Tagg266 (protein aggregation temperature).

**Table 2. Thermal stability of different Fc variants**

| **Sample name** | Tonset(°C) | **Tm1 (°C)** | Tm2 (°C) | Tm3 (°C) | Tagg266 (°C) | Tagg473 (°C) |
|---|---|---|---|---|---|---|
| Wild-type | 65.49 | 71.20 | | 85.50 | 71.72 | 87.87 |
| Wild-type-fucose- | 65.50 | 70.98 | 83.20 | - | 70.23 | 31.43 |
| Antibody 1 | 60.57 | 68.06 | 77.87 | 82.90 | 70.18 | 75.87 |
| Antibody 2 | 64.74 | 68.93 | 74.20 | 79.40 | 69.97 | 76.38 |
| Antibody 3 | 57.62 | 60.57 | 69.13 | 80.10 | 70.40 | 32.41 |
| Antibody 5 | 48.25 | 50.71 | 69.00 | 81.14 | 70.54 | 49.06 |

The results showed (Table 2) that each antibody had a Tm value above 50°C. The Tm values of antibodies 1, 2 and 3 above 60°C were more stable. G237A alone resulted in a negligible change in Tm.

### Example 5. ADCP Activity Assay

Antibody 8, antibody 7, and the control were diluted 5-fold from 10 µg/ml for 7 concentration points. Romas cells were labeled with CFSE. Monocyte cells were stimulated to obtain MΦ and plated at 8 × 10⁵ cells/well (50 µl). 50 µl of the diluted sample was added to MΦ and incubated at 37°C in a 5% CO₂ incubator for 15 min. Then, the labeled Romas cells were added at a ratio of 1: 5 (MΦ cell amount of 8 × 10⁵ cells/well, Romas cell amount of 4 × 10⁶ cells/well), plated on a 48-well cell plate, and incubated at 37°C in a 5% CO₂ incubator for 2.5 h. The flow cytometry was performed after CD11b-APC staining.

The results showed (FIG. 3) that antibody 8 and antibody 7 had a strong phagocytic effect. This indicates that the mutation cannot adversely affect the ADCP activity of the antibody.

### Example 6. CDC Activity Assay

Antibody 3 was diluted 9 times with a 1640 medium containing 0.5% BSA starting at 100 µg/ml in 5-fold ratios to obtain 10 concentration points. The diluted sample was added at 40 µl/well to a 96-well cell plate (5 × 10⁴ cells/well) plated with 30 µl of Daudi cells. The cell plate was incubated at 4°C for 1 hr. Normal human serum was diluted 2-fold with 0.5% BSA in the 1640 medium, added into sample wells at 40 µl/well, and incubated at 37°C for 2hr. The viability of the remaining cells was detected by Celltiter-Glo^{™}.

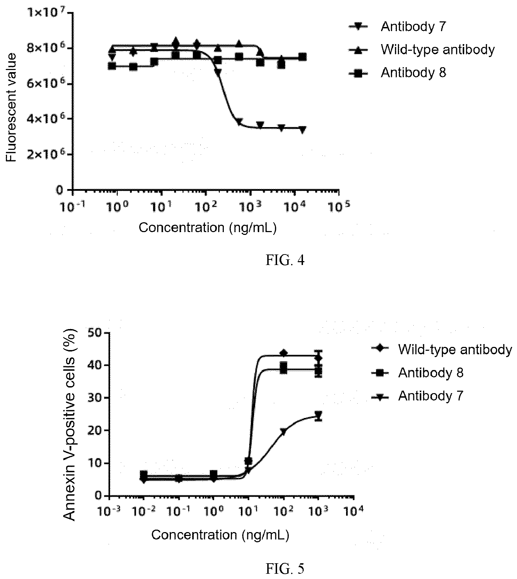

The results show (FIG. 4) that antibody 8 had no CDC activity as did the wild-type antibody, and that the mutation cannot change the CDC activity profile. The other antibody 7 retained CDC activity, consistent with the reported wild-type antibody (Michael Karl Bauer, CD38: A Unique Target In Multiple Myeloma, 2014).

### Example 7. Assay of The Ability to Induce Apoptosis

Daudi cells were seeded to a plate. After adjusting the density to 750 µl/well (2 × 10⁵ cells/well), the cells were plated to a 24-well plate. The antibody 3 to be tested was diluted to 4 µg/ml with 1640 added with 10% serum (Sigma-Aldrich) and 2 mM L-glutamine and further diluted 5 times in a 10-fold ratio to obtain samples with 6 concentration gradients. 250 µl of diluted sample was added to cells of the 24-well plate, with the 1640 medium as a negative control (NC). The 24-well plate was placed in an incubator containing 5% CO₂ at 37°C for 20 h. After completion, the cells were transferred to a 1.5 ml EP tube and centrifuged at 1000 rpm for 5 min. The supernatant was discarded. The cells were added with 200 µl of PBS to be resuspended, transferred to a 96-well tip plate, and centrifuged to discard the supernatant. After staining and incubating with Annexin V-FITC kit for 10-20 minutes, flow cytometry detection was performed, and the proportion of apoptotic cells was analyzed using GraphPad Prism.

The results showed (FIG. 5) that antibody 8 induced apoptosis close to that of the wild-type antibody. The mutation cannot affect the ability to induce apoptosis. Another antibody 7 retained the ability to induce apoptosis.

### Example 8. Binding Activity Assay

The affinity of wild-type antibody and antibody 3 to the target CD38 and to human FcRn under acidic conditions was determined by BLI method. The results showed (Table 3) that the affinity of antibody 8 to both proteins was within 3-fold that of the wild-type antibody to CD38, indicating that the mutation cannot affect the binding activity of Fab.

**Table 3. Binding activity assay**

| **Comparison item** | **Wild-type antibody** | **Antibody 8** |
|---|---|---|
| hFcRn (KD) | 5.80E-08 | 1.96E-08 |
| CD38 (KD) | 2.75E-10 | 1.22E-10 |

### Example 9. Inhibition enzyme activity assay

Antibody 8 and the wild-type antibody were diluted to 40 nM and CD38 protein to 10 nM, respectively, with 20 mM Tris-HCl, pH 7.0. The diluted CD38 was taken and mixed with the antibody at the ratio of 1: 1. Then, the diluent without the antibody was used as a blank control and the reaction was carried out at room temperature protected from light for 15 min. After completion of the reaction, 100 µl of NGD (nicotinamide guanine dinucleotide sodium salt) at a final concentration of 80 µM was added and incubated at room temperature, protected from light, and detected at 60 min after incubation. The enzyme activity inhibition effect was determined by measuring the fluorescence signal at the excitation wavelength of 300 nm and the emission wavelength of 410 nm. The lower the RFU value, the higher the inhibitory effect.

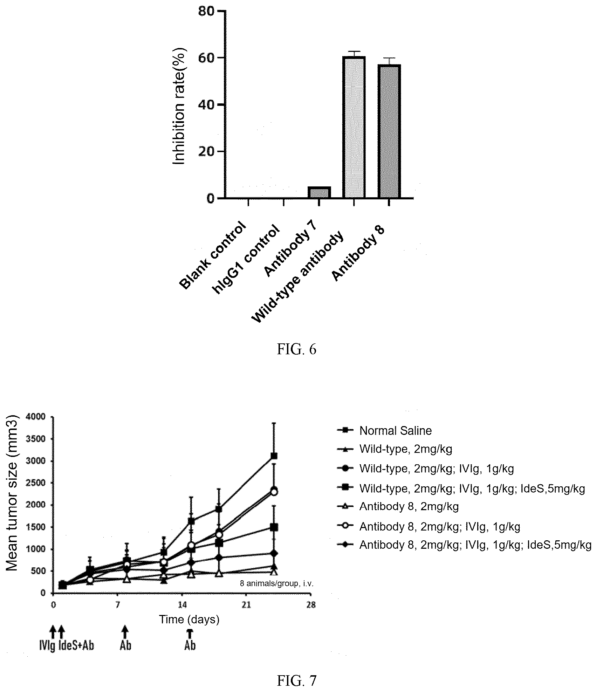

The results (FIG. 6) showed that antibody 8 was essentially identical to the wild-type antibody in its ability to inhibit CD38 enzymatic activity, indicating that this mutation cannot affect the inhibitory function of Fab on CD38 enzymatic activity. Antibody 7 had little inhibitory activity.

### Example 10. Animal efficacy

*In vivo* activity of the variants of the invention was assessed using the Daudi mouse model. The experiment was designed as 7 groups, with groups 1 to 7 going from top to bottom as shown in FIG. 7. Specifically, logarithmic growth phase cells were taken. 5 × 10⁶ cells of Daudi cells were subcutaneously inoculated into the right axilla of each huPBMC-NOG-Dko mouse under sterile conditions, with an inoculation amount of 0.1 mL per mouse. About 10 days after cell inoculation, when tumor volume grew to about 200 mm³, 56 animals were randomly divided into 7 groups (8 animals per group) according to tumor volume screening. On the day of grouping, animals in groups 3, 4, 6, and 7 were given a single intraperitoneal injection of IVIg (volume: 20 mL/kg, dose: 1 g/kg); 24 h later, animals in groups 4 and 7 were given a single intravenous injection of IdeS (volume: 10 mL/kg, dose: 5 mg/kg). The antibody (volume: 10 mL/kg, dose: 2 mg/kg) was administered intravenously 2 h after IdeS administration. The day of the first administration of the antibody was recorded as D1. Animals in groups 2-7 were intravenously injected with the antibody at D8 and D15. The vehicle control group was intravenously injected with the corresponding volume of the normal saline. After administration, tumor volume was measured twice a week.

The results of the experiment (FIG. 7) show that the administration of antibody 8 alone was as effective as the administration of the wild-type antibody alone. The presence of IgG (IVIg) inhibited the efficacy of the antibody in the treatment of tumors. The combination of antibody 8 and immunoglobulin degrading enzyme can reduce the inhibition of IgG on antibody efficacy and significantly enhance the therapeutic effect of therapeutic antibodies. The wild antibody didn't have this effect.

The applicant declares that the present invention illustrates the detailed methods of the present invention through the above embodiments, but the present invention is not limited to the above-detailed methods, which does not mean that the present invention must rely on the above-detailed methods to be implemented. It will be apparent to those skilled in the art that any modifications to the present invention, equivalent alterations to the various raw materials of the products of the present invention, and additions of auxiliary components, selections of specific ways, etc. fall within the scope and disclosure of the present invention.

**SEQUENCE LISTING**

| **SEQ ID NO:** | **Sequence Description** | **SEQ** |
|---|---|---|
| 1 | HCDR1 | SFAMS |
| 2 | HCDR2 | AISGSGGGTYYADSVK |
| 3 | HCDR3 | DKILWFGEPVFDY |
| 4 | HCDR1 | DYWMQ |
| 5 | HCDR2 | TIYPGDGDTGYAQKFQ |
| 6 | HCDR3 | GDYYGSNSLDY |
| 7 | LCDR1 | RASQSVSSYLA |
| 8 | LCDR2 | DASNRAT |
| 9 | LCDR3 | QQRSNWPPTF |
| 10 | LCDR1 | KASQDVSTVVA |
| 11 | LCDR2 | SASYRYI |
| 12 | LCDR3 | QQHYSPPYT |
| 13 | VH | |
| 14 | VH | |
| 15 | VL | |
| 16 | VL | |
| 17 | G1M1 | LGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLY SKLTVDKSRWQQGNVFSCSVMHEALHNHYTQKSLSLSP |
| 18 | G1M3, 17 | LGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSP |
| 19 | L235-K340 | LGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK |
| 20 | Heavy Chain Wild-type | QVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 21 | Heavy Chain 1 | QVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLGAPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 22 | Heavy Chain 2 | QVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLGAPDVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 23 | Heavy Chain 3 | QVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLGAPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPEEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 24 | Heavy Chain 4 | QVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLGGPDVFLFPPKPKDTLMISRTPEVTCVVVDVSHEDPEVKFNWYVDGVEVH NAKTKPREEQYNSTYRVVSVLTVLHQDWLNGKEYKCKVSNKALPAPEEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 25 | Heavy Chain 5 | QVQLVQSGAEVAKPGTSVKLSCKASGYTFTDYWMQWVKQRPGQGLEWIGTIYPGDG |
| | | DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLGAPDVFLFPPKPKDTLMISRTPEVTCVVVDVSHEDPEVKFNWYVDGVEVH NAKTKPREEQYNSTYRVVSVLTVLHQDWLNGKEYKCKVSNKALPAPEEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 26 | Heavy Chain 6 | QVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQ GTSVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCP PCPAPELLAGPDVFLFPPKPKDTLMISRTPEVTCVVVDVSHEDPEVKFNWYVDGVEVH NAKTKPREEQYNSTYRVVSVLTVLHQDWLNGKEYKCKVSNKALPAPEEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSP |
| 27 | Light Chain 1 | DIVMTQSHLSMSTSLGDPVSITCKASQDVSTVVAWYQQKPGQSPRRLIYSASYRYIGVP DRFTGSGAGTDFTFTISSVQAEDLAVYYCQQHYSPPYTFGGGTKLEIKRTVAAPSVFIFP PSDEQLKSGTASVVCLLNNFYPREAKVQWKVDNALQSGNSQESVTEQDSKDSTYSLS STLTLSKADYEKHKVYACEVTHQGLSSPVTKSFNRGEC |
| 28 | Light Chain 2 | EIVLTQSPATLSLSPGERATLSCRASQSVSSYLAWYQQKPGQAPRLLIYDASNRATGIPA RFSGSGSGTDFTLTISSLEPEDFAVYYCQQRSNWPPTFGQGTKVEIKRTVAAPSVFIFPPS DEQLKSGTASVVCLLNNFYPREAKVQWKVDNALQSGNSQESVTEQDSKDSTYSLSST LTLSKADYEKHKVYACEVTHQGLSSPVTKSFNRGEC |
| 29 | Heavy Chain 7 | EVQLLESGGGLVQPGGSLRLSCAVSGFTFNSFAMSWVRQAPGKGLEWVSAISGSGGGT YYADSVKGRFTISRDNSKNTLYLQMNSLRAEDTAVYFCAKDKILWFGEPVFDYWGQG TLVTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKRVEPKSCDKTHTCPP CPAPELLGAPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPEEKTISKAKGQP REPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSP |
| 30 | Heavy Chain 8 | EVQLLESGGGLVQPGGSLRLSCAVSGFTFNSFAMSWVRQAPGKGLEWVSAISGSGGGT YYADSVKGRFTISRDNSKNTLYLQMNSLRAEDTAVYFCAKDKILWFGEPVFDYWGQG TLVTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKRVEPKSCDKTHTCPP CPAPELLGAPDVFLFPPKPKDTLMISRTPEVTCVVVDVSHEDPEVKFNWYVDGVEVHN AKTKPREEQYNSTYRVVSVLTVLHQDWLNGKEYKCKVSNKALPAPEEKTISKAKGQP REPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSP |
| 31 | IdeS | MDSFSANQEIRYSEVTPYHVTSVWTKGVTPPANFTQGEDVFHAPYVANQGWYDITKT FNGKDDLLCGAATAGNMLHWWFDQNKDQIKRYLEEHPEKQKINFNGEQMFDVKEAI DTKNHQLDSKLFEYFKEKAFPYLSTKHLGVFPDHVIDMFINGYRLSLTNHGPTPVKEG SKDPRGGIFDAVFTRGDQSKLLTSRHDFKEKNLKEISDLIKKELTEGKALGLSHTYANV RINHVINLWGADFDSNGNLKAIYVTDSDSNASIGMKKYFVGVNSAGKVAISAKEIKED NIGAQVLGLFTLSTGQDSWNQTN |
| 32 | Signal peptide | MRKRCYSTSAAVLAAVTLFVLSVDRGVIA |
| 33 | CH1 | ASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKV |
| 34 | Hinge | EPKSCDKTHTCPPCP |
| 35 | CH2 | APELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAK |
| 36 | CH3 | GQPREPQVYTLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |

## Claims

1. A molecule comprising an Fc variant, wherein compared with a molecule comprising an IgG wild-type Fc, the molecule is resistant to the degradation of an immunoglobulin enzyme, and the molecule comprises a human IgG Fc region with a mutation, the mutation being one or more substitutions or deletions at a G237 position (EU numbering), and the substitution preferably being G237A; preferably, the human IgG Fc region comprises at least a sequence as shown in SEQ ID NO: 19.

2. The molecule of claim 1, wherein the Fc molecule is resistant to the degradation of a protease capable of cleaving a molecule comprising an IgG wild-type Fc between residues 236-237 (EU numbering).

3. The molecule of claim 1 or 2, wherein compared with the wild-type IgG, the molecule is resistant to the degradation of a protease selected from the group consisting of IdeS, IdeZ, IdeE, IdeZ2, IdeE2, IdeSORK, Xork, and variants thereof.

4. The molecule of any one of claims 1-3, wherein the IgG of the molecule is selected from the group consisting of IgG1, IgG2, IgG3, and IgG4.

5. The molecule of any one of claims 1-4, wherein compared with a molecule comprising an IgG wild-type Fc, the molecule has promoted or enhanced effector functions selected from the group consisting of ADCC, ADCP, and CDC.

6. The molecule of claim 5, further comprising one or more substitutions in the human IgG wild-type Fc region sequence selected from the group consisting of S239D, I332E, G236A, S298A/E333A/K334A, S239D/I332E, S239D/A330L/I332E, G236A/S239D/I332E, G236A/A330L/I332E, G236A/S239D/A330L/I332E, F243L/R292P/Y300L, F243L/R292P/Y300L/V305I/P396L, L235V/F243L/R292P/Y300L/P396L, P247I/A339Q, K326A/E333A, K326W/E333S, E333A/K334A, H268F/S324T/I332E, S239D/H268F/S324T/I332E, S267E/H268F/S324T/I332E, K326A/I332E/E333A, S239D/K326A/E333A and S267E/I332E; preferably, the substitution is selected from the group consisting of S239D, I332E and S239D/I332E.

7. The molecule of any one of claims 1-6, further comprising a N-glycan modification having a fucose content of less than 50%, preferably less than 10%.

8. The Fc molecule of any one of claims 1-7, further comprising a N-glycan modification having a terminal galactose modification content of higher than 30%, preferably higher than 50%.

9. The molecule of any one of claims 1-8, wherein the molecule is an antibody or Fc fusion protein.

10. The molecule of claim 9, wherein the antibody binds to an antigen on a virus, bacterium, tumor cell, tumor stroma, or tumor vasculature, preferably the antibody specifically binds to one or two antigens selected from the group consisting of COVID-19, CD38, CD20, FR5, BCMA, SLAM7, CD73, GPRCD5, and CD3, most preferably, the antibody specifically binds to CD38.

11. The molecule of claim 9 or 10, wherein the antibody comprises at least one heavy chain and at least one light chain, wherein the heavy chain comprises HCDR1 as shown in SEQ ID NO: 1, HCDR2 as shown in SEQ ID NO: 2, and HCDR3 as shown in SEQ ID NO: 3; or, HCDR1 as shown in SEQ ID NO: 4, HCDR2 as shown in SEQ ID NO: 5, and HCDR3 as shown in SEQ ID NO: 6; wherein the light chain comprises LCDR1 as shown in SEQ ID NO: 7, LCDR2 as shown in SEQ ID NO: 8, and LCDR3 as shown in SEQ ID NO: 9; or, LCDR1 as shown in SEQ ID NO: 10, LCDR2 as shown in SEQ ID NO: 11, and LCDR3 as shown in SEQ ID NO: 12.

12. The molecule of any one of claims 9-11, wherein the antibody comprises at least one heavy chain and at least one light chain, wherein the heavy chain comprises a heavy chain variable region as shown in an amino acid sequence of SEQ ID NO: 13 and the light chain comprises a light chain variable region as shown in an amino acid sequence of SEQ ID NO: 14; or wherein the heavy chain comprises a heavy chain variable region as shown in an amino acid sequence of SEQ ID NO: 15 and the light chain comprises a light chain variable region as shown in an amino acid sequence of SEQ ID NO: 16.

13. The molecule of any one of claims 9-12, wherein an epitope-binding fragment of the antibody is Fab, Fab' and F(ab')2, scFv or a disulfide-linked Fv fragment.

14. The molecule of any one of claims 1-4, wherein compared with a molecule comprising an IgG1wild-type Fc, the molecule has weakened and/or eliminated effector functions selected from the group consisting of ADCC, ADCP, and CDC.

15. The Fc molecule of claim 14, further comprising one or more substitutions in the human IgG1 wild-type Fc sequence selected from the group consisting of N297A, N297Q, N297G, L235A, L235A/E318A, L234A/L235A, G236R/L328R, S298G/T299A, L234F/L235E/P331S, E233P/L234V/L235A/G236del/S267K, L234A/L235A/P329G, and L234F/L235E/D265A; preferably, the substitution is selected from the group consisting of N297A, N297Q, N297G, L234A/L235A, L235A, and L234F/L235E/P331S.

16. The molecule of any one of claims 1-15, wherein compared with a molecule comprising an IgG1 wild-type Fc, the molecule has increased affinity to FcRn.

17. The molecule of claim 16, further comprising one or more substitutions in the sequence of IgG1wild-type Fc selected from the group consisting of YTEKF, YTEKF+V264A, and YTEKF+V264E.

18. An isolated binding polypeptide molecule comprising: (i) a binding domain capable of binding to a target molecule on or bound to a cell, and (ii) an Fc domain having amino acid sequences of CH2 and CH3 constant domains of a human IgG1heavy chain, with residue G237 as defined by the EU numbering system being substituted or deleted, **characterized in that** the binding molecule is capable of binding to a target molecule on a target cell and the molecule produces a measurable CDC activity, or a necessary effector cell-mediated target cell damaging activity, preferably wherein the substitution at G237 is G237A.

19. An immunoconjugate comprising the molecule of any one of claims 1 to 17 or the binding polypeptide molecule of claim 18, and a cytotoxic agent.

20. An isolated nucleic acid or isolated nucleic acids, encoding the molecule of any one of claims 1-17 or the binding polypeptide molecule of claim 18.

21. An expression vector containing a nucleic acid of claim 20.

22. A host cell comprising the nucleic acid of claim 20 or the expression vector of claim 21.

23. A method for preparing the molecule of any one of claims 1-17 or the binding polypeptide molecule of claim 18, comprising:
(a) culturing the host cell of claim 22 under conditions suitable for the expression of the molecule or the binding polypeptide molecule; and
(b) optionally recovering the molecule or the binding polypeptide molecule from the culture of step (a).

24. A molecule or binding polypeptide molecule produced by the method of claim 23.

25. A pharmaceutical composition comprising the molecule of any one of claims 1 to 17, the binding polypeptide molecule of claim 18, or the immunoconjugate of claim 19, and an immunoglobulin degrading enzyme capable of cleaving an IgG molecule between residues 236-237 (EU numbering); wherein the immunoglobulin degrading enzymes are preferably IdeS, IdeZ, IdeE, IdeSORK, Xork, and variants of these enzymes.

26. A pharmaceutical composition comprising the molecule of any one of claims 1 to 17, the binding polypeptide molecule of claim 18, or the immunoconjugate of claim 19, and a pharmaceutical carrier.

27. The molecule of any one of claims 1-17, the binding polypeptide molecule of claim 18, the immunoconjugate of claim 19, or the pharmaceutical composition of any one of claims 25-26, used as a drug; preferably the drug is for treating a disease; more preferably, the disease is cancer or autoimmune disease; most preferably, wherein the cancer is multiple myeloma.

28. Use of the molecule of any one of claims 1-17, the binding polypeptide molecule of claim 18, the immunoconjugate of claim 19, or the pharmaceutical composition of any one of claims 25-26 in the preparation of a drug for the treatment of diseases, particularly cancer or autoimmune diseases.

29. The use of the molecule of any one of claims 1-17, the binding polypeptide molecule of claim 18, the immunoconjugate of claim 19, or the pharmaceutical composition of any one of claims 25-26 in the preparation of a drug for inducing ADCC, ADCP, and apoptosis-mediated depletion of CD38 positive cells.

30. A method of treating cancer or autoimmune disease, comprising administering to a subject an effective amount of the molecule of any one of claims 1-17, the binding polypeptide molecule of claim 18, the immunoconjugate of claim 19, or the pharmaceutical composition of any one of claims 25-26.

31. A method of ADCC, ADCP-mediated depletion of CD38 positive cells in a subject, comprising administering to the subject an effective amount of the molecule of any one of claims 1-17, the binding polypeptide molecule of claim 18, the immunoconjugate of claim 19, or the pharmaceutical composition of any one of claims 25-26 to induce ADCC, ADCP, apoptosis-mediated depletion of CD38 positive cells.

32. The invention as described herein.
